(19) Europäisches Patentamt
European Patent Office
Office européen des brevets

(11) **EP 3 960 847 A1**

(12) **EUROPEAN PATENT APPLICATION**
published in accordance with Art. 153(4) EPC

(43) Date of publication:
**02.03.2022 Bulletin 2022/09**

(21) Application number: **20796207.7**

(22) Date of filing: **24.04.2020**

(51) International Patent Classification (IPC):
**C12N 5/07** (2010.01)        **C12N 1/04** (2006.01)

(52) Cooperative Patent Classification (CPC):
**C12N 1/04**

(86) International application number:
**PCT/JP2020/017586**

(87) International publication number:
**WO 2020/218461 (29.10.2020 Gazette 2020/44)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.04.2019   JP 2019085499**

(71) Applicant: **Otsuka Pharmaceutical Factory, Inc.
Naruto-shi, Tokushima 772-8601 (JP)**

(72) Inventors:
• **HASHIMOTO, Kazumasa
Naruto-shi, Tokushima 772-8601 (JP)**
• **NISHIMURA, Masuhiro
Naruto-shi, Tokushima 772-8601 (JP)**

• **FUJITA, Yasutaka
Naruto-shi, Tokushima 772-8601 (JP)**
• **TADA, Akihiro
Naruto-shi, Tokushima 772-8601 (JP)**
• **TSUBAKIYAMA, Ryohei
Naruto-shi, Tokushima 772-8601 (JP)**
• **ONODERA, Kyoka
Naruto-shi, Tokushima 772-8601 (JP)**
• **NOMURA, Yoshiki
Naruto-shi, Tokushima 772-8601 (JP)**
• **SHIRAKAWA, Chikage
Naruto-shi, Tokushima 772-8601 (JP)**

(74) Representative: **Hamer, Christopher K. et al
Mathys & Squire
The Shard
32 London Bridge Street
London SE1 9SG (GB)**

(54) **TREHALOSE-CONTAINING LIQUID FOR MAMMALIAN CELL PRESERVATION**

(57)    The present invention addresses the problem of providing, for instance, a mammalian cell preservation solution that can effectively suppress a decrease in cell viability occurring when mammalian cells are preserved in liquid or a decrease in self-renewal potential occurring when mammalian stem cells are preserved in liquid, and that is less likely to cause a harmful effect on the life of a mammal at the time of *in vivo* administration of mammalian cells to the mammal. This solution involves preserving a mammalian cell by using a mammalian cell preservation solution comprising trehalose or a derivative thereof, or a salt thereof and a hydrogen carbonate, such as sodium bicarbonate, as a pH modifier, and having a pH of from 6.5 to 8.5.

**EP 3 960 847 A1**

## Description

## Technical Field

The present invention relates to a mammalian cell preservation solution (hereinafter, sometimes referred to as "the present mammalian cell preservation solution") comprising trehalose or a derivative thereof, or a salt thereof (hereinafter, sometimes generally referred to as a "trehalose group") and having a hydrogen-ion exponent (pH) of 6.5 to 8.5, a method for preserving a mammalian cell using the present mammalian cell preservation solution, and the like.

## Background Art

In recent years, there has been a growing interest in regenerative medicine due to the rapid development of stem cell research. The knowledge and understanding are becoming more widespread not only among researchers but also among the general public. Stem cell-based regenerative medicine is medicine aiming to restore the functions of cells and/or tissues damaged by various diseases by utilizing the self-renewal and pluripotent differentiation potentials of stem cells and/or the factors secreted by stem cells. When bone marrow transplantation is performed on patients with intractable hematological diseases such as leukemia or aplastic anemia, hematopoietic stem cells can be engrafted in the patient's body and hematopoiesis can be maintained for almost a lifetime. In addition, many researchers are now aiming to use stem cells other than hematopoietic stem cells for clinical applications, and have identified stem cells in the central nervous system, peripheral nerves, bone marrow, small intestine, and the like. Then, tissue stem cell transplantation therapy for traumatic or tissue degenerative diseases start to be implemented (Non-Patent Documents 1 to 3). On the other hand, cancer immuno-cell therapy is a state-of-the-art cellular medicine in which immune cells that function to attack cancer are removed from the body, their functions are strengthened, and the cells are then returned to the body. T-cell-based therapies such as dendritic cell vaccine therapy, alpha-beta T-cell therapy ($\alpha\beta$T-cell therapy), gamma-delta T-cell therapy ($\gamma\delta$T-cell therapy), CTL therapy, and natural killer cell therapy (NK cell therapy) are being in practice.

When stem cells or T cells used for transplantation therapy are preserved for a long period, favorable cell viability cannot be maintained by preservation in liquid. For example, it has been reported that when human bone marrow stem cells are refrigerated (at 4°C) and preserved in saline, the cell viability decreases to 40% or less after 48 h and decreases to 20% or less after 72 h (Non-Patent Document 4). For this reason, cryopreservation is commonly used when stem cells for transplantation or T cells for transplantation are preserved for a long period. However, cryopreservation solutions usually each contain a cryopreservative such as DMSO and/or glycerol. Thus, after thawing the cryopreserved stem cells or T cells, it is necessary to remove the cryopreservative before transplantation therapy. It takes a lot of time and effort. This has been a problem. In addition, even if a cryopreservative is added to a cryopreservation solution, the cytoskeleton is severely damaged by water crystallization during freezing. This has unfortunately caused a decrease in cell viability after freeze-thawing. In view of the above, there is an urgent need for the development of a cell preservation solution that is easy to use and can suppress the decrease in cell viability.

The present inventors have reported that trehalose exerts a suppressive action on a decrease in cell viability occurring when mammalian cells are preserved in liquid (Patent Documents 1 and 2). However, it has been unknown that when the pH of the above liquid is adjusted, the suppressive effect on a decrease in cell viability can be further enhanced or the suppressive effect on a decrease in self-renewal potential of mammalian stem cells is exerted.

## Prior Art Documents

## Patent Documents

Patent Document 1: Japanese unexamined Patent Application Publication No. 2012-115253
Patent Document 2: International Publication No. WO 2014/208053

## Non-Patent Documents

Non-Patent Document 1: Gage, F.H. Science 287: 1433-1438 (2000)
Non-Patent Document 2: Morrison, S.J. et al., Cell 96: 737-749 (1999)
Non-Patent Document 3: Batle, E. et al., Cell 111: 251-263 (2002)

Non-Patent Document 4: Lane, T.A. et al., Transfusion 49: 1471-1481 (2009)

## Summary of the Invention

### Object to be Solved by the Invention

An object of the present invention is to provide, for instance, a mammalian cell preservation solution that can effectively suppress a decrease in cell viability occurring when mammalian cells are preserved in liquid or a decrease in self-renewal potential occurring when mammalian stem cells are preserved in liquid, and that is less likely to cause a harmful effect on the life of a mammal at the time of *in vivo* administration of mammalian cells to the mammal.

### Means to Solve the Object

The present inventors have conducted intensive research to solve the above problem. It has been found during the course that when mammalian cells are preserved in a solution comprising a trehalose group and a hydrogen carbonate as a pH modifier and having a pH of from 6.5 to 8.5, cell death is effectively suppressed and the percentage of viable cells can be increased even in the case where the solution has no pH buffering action. It has also been demonstrated that when mammalian stem cells are preserved in the solution, a decrease in self-renewal potential of the mammalian stem cells can be effectively suppressed even in the case where the solution has no pH buffering action. Based on these findings, the present invention has been completed.

Specifically, the present invention is as follows.

[1] A mammalian cell preservation solution comprising trehalose or a derivative thereof, or a salt thereof and a hydrogen carbonate as a pH modifier, and having a pH of 6.5 to 8.5.

[2] The preservation solution according to [1], wherein the hydrogen carbonate is sodium bicarbonate.

[3] The preservation solution according to [1] or [2], further comprising a polysaccharide or a derivative thereof, or a salt thereof.

[4] The preservation solution according to any one of [1] to [3], wherein the preservation solution is an isotonic solution.

[5] The preservation solution according to [4], wherein the isotonic solution is a lactated Ringer's solution.

[6] The preservation solution according to any one of [3] to [5], wherein the polysaccharide is dextran.

[7] The preservation solution according to any one of [1] to [6], wherein a concentration of the trehalose or derivative thereof, or salt thereof is 2.0 to 6.0% (w/v).

[8] The preservation solution according to [6] or [7], wherein a concentration of the dextran or derivative thereof, or salt thereof is 4.0 to 7.0% (w/v).

[9] The preservation solution according to any one of [1] to [8], wherein the preservation solution is for preserving a mammalian cell at 0 to 40°C.

[10] The preservation solution according to any one of [1] to [9], wherein the preservation solution is for preserving a mammalian cell for a period of 6 h to 14 days.

[11] The preservation solution according to any one of [1] to [10], wherein the preservation solution is used for suppressing a decrease in viability of a mammalian cell.

[12] The preservation solution according to any one of [1] to [11], wherein the preservation solution is used for suppressing a decrease in self-renewal potential of a mammalian cell.

[13] The preservation solution according to any one of [1] to [12], wherein the preservation solution is used for mammalian cell transplantation.

[14] The preservation solution according to any one of [1] to [13], wherein the mammalian cell is a mammalian stem cell.

[15] The preservation solution according to [14], wherein the mammalian stem cell is a mammalian mesenchymal stem cell.

[16] A powder formulation comprising trehalose or a derivative thereof, or a salt thereof and a hydrogen carbonate as a pH modifier, for preparing the preservation solution according to any one of [1] to [15].

[17] The powder formulation according to [16], wherein the hydrogen carbonate is sodium bicarbonate.

[18] A method of preserving a mammalian cell, comprising a step of preserving a mammalian cell in a preservation solution comprising trehalose or a derivative thereof, or a salt thereof and a hydrogen carbonate as a pH modifier, and having a pH of 6.5 to 8.5.

[19] The method of preserving a mammalian cell according to [18], wherein the hydrogen carbonate is sodium bicarbonate.

[20] The method of preserving a mammalian cell according to [18] or [19], wherein the preservation solution further comprises a polysaccharide or a derivative thereof, or a salt thereof.

[21] The method of preserving a mammalian cell according to any one of [18] to [20], wherein the preservation solution is an isotonic solution.

[22] The method of preserving a mammalian cell according to [21], wherein the isotonic solution is a lactated Ringer's solution.

[23] The method of preserving a mammalian cell according to any one of [20] to [22], wherein the polysaccharide is dextran.

[24] The method of preserving a mammalian cell according to any one of [18] to [23], wherein the mammalian cell is preserved in the preservation solution for 6 h to 14 days.

Further, examples of another embodiment of the present invention include: a method of transplanting a mammalian cell, comprising the step of administering, to a subject in need of mammalian cell transplantation (e.g., a patient with traumatic disease, a patient with tissue degenerative disease, a cancer patient), the present mammalian cell preservation solution comprising a mammalian cell; a method of transplanting a mammalian cell, comprising the steps of preparing the present mammalian cell preservation solution by adding a mammalian cell to a trehalose group-containing solution (preferably an isotonic solution) or adding a trehalose group to a mammalian cell-containing solution (preferably an isotonic solution) and further adjusting the solution to pH 6.5 to 8.5, preserving the mammalian cell in the present mammalian cell preservation solution prepared, and administering, to a subject in need of mammalian cell transplantation (e.g., a patient with traumatic disease, a patient with tissue degenerative disease, a cancer patient), the present mammalian cell preservation solution comprising the preserved mammalian cell; use of a trehalose group in the manufacture of the present mammalian cell preservation solution or use of a trehalose group for suppressing a decrease in mammalian cell viability in solution; the present mammalian cell preservation solution further comprising a mammalian cell for use in treatment of a disease (e.g., traumatic disease, tissue degenerative disease, cancer) in need of mammalian cell transplantation therapy; a method of preparing the present mammalian cell preservation solution comprising a mammalian cell, comprising the steps of preparing the present mammalian cell preservation solution by adding a mammalian cell to a trehalose group-containing liquid (preferably an isotonic solution) or adding a trehalose group to a mammalian cell-containing solution (preferably an isotonic solution) and further adjusting the solution to pH 6.5 to 8.5; and the present mammalian cell preservation solution further comprising a mammalian cell. Note that the step of preserving a mammalian cell in the above transplantation method is to keep the present mammalian cell preservation solution comprising a mammalian cell under temperature conditions in which the preservation solution is present in a liquid state, and does not include a step of keeping the preservation solution in a solid state (e.g., a step of preserving a mammalian cell in a dormant state, such as a cryopreservation step or a lyophilization preservation step).

**Effect of the Invention**

According to the present invention, addition of a trehalose group to a liquid and adjustment of the pH of the liquid to 6.5 to 8.5 make it possible to effectively suppress a decrease in cell viability occurring when mammalian cells are preserved in liquid and/or a decrease in self-renewal potential occurring when mammalian stem cells are preserved in liquid. Further, the trehalose group is a disaccharide that is less likely to cause a harmful effect on the life of a mammal in the case of *in vivo* administration to the mammal. This makes it possible to *in vivo* administer to the mammal, without replacement by a fresh liquid for transplantation, the mammalian cells as they are after they are preserved in the present mammalian cell preservation solution.

**Brief Description of Drawings**

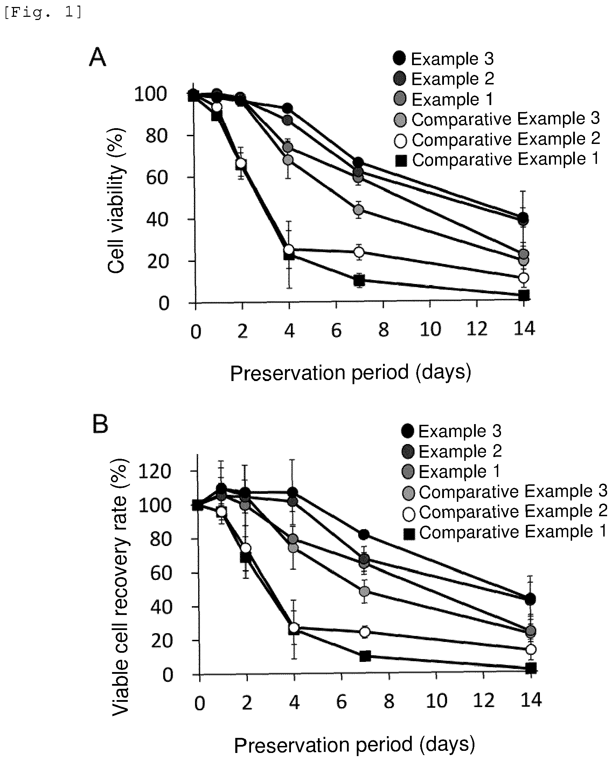

[Figure 1] Figure 1 is graphs showing the results of measuring the cell viability (Figure 1A) and the viable cell recovery rate (Figure 1B) when human Adipose tissue-derived Mesenchymal Stem Cells (hAD-MSC) were preserved in trehalose-containing preservation solution CSP-01 for each preservation period (1 day, 2 days, 4 days, 7 days, or 14 days) in Examples 1 to 3 or Comparative Examples 1 to 3.

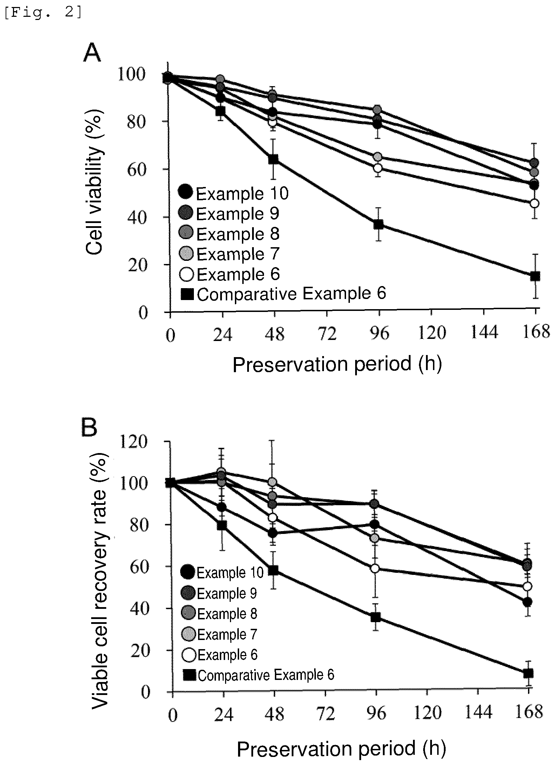

[Figure 2] Figure 2 is graphs showing the results of measuring the cell viability (Figure 2A) and the viable cell recovery rate (Figure 2B) when hAD-MSC were preserved in trehalose-containing preservation solution CSP-01 for each preservation period (24 h, 48 h, 96 h, or 168 h) in Examples 6 to 10 or Comparative Example 6.

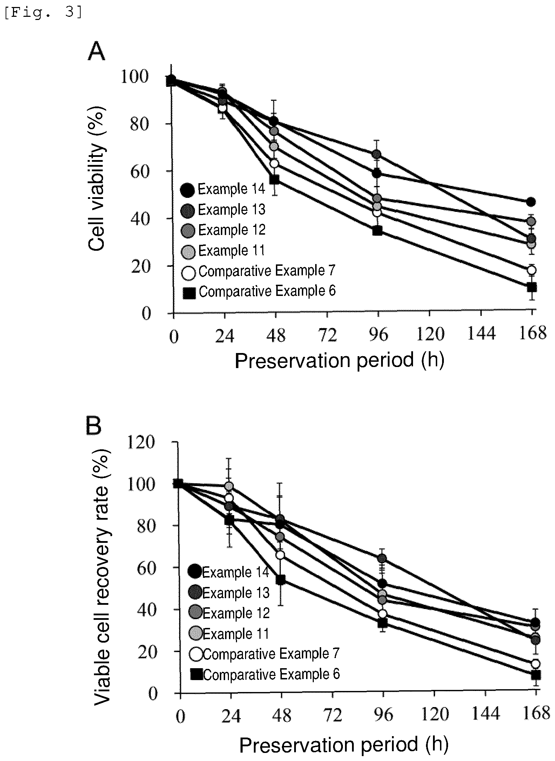

[Figure 3] Figure 3 is graphs showing the results of measuring the cell viability (Figure 3A) and the viable cell recovery rate (Figure 3B) when hAD-MSC were preserved in trehalose-containing preservation solution CSP-01 for each preservation period (24 h, 48 h, 96 h, or 168 h) in Examples 11 to 14 or Comparative Example 6 or 7.

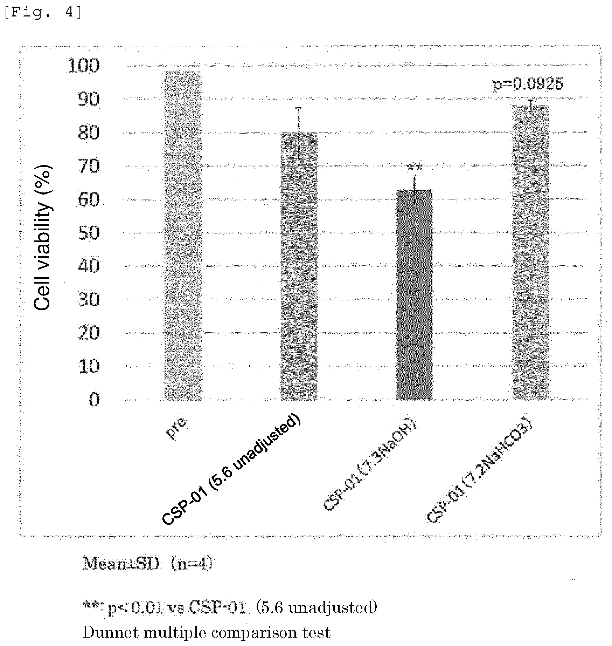

[Figure 4] Figure 4 is a graph showing both the pre-preservation (pre) measurement results and the results of measuring the cell viability after Human Bone Marrow Mesenchymal Stem Cells (hBM-MSC) were preserved at 5°C for 3 days in trehalose-containing preservation solution CSP-01 (pH 5.6 unadjusted), CSP-01 (pH7.3 NaOH), or CSP-01 (7.2 NaHCO$_3$).

**Mode of Carrying Out the Invention**

A mammalian cell preservation solution of the present invention is a solution (i.e., the present mammalian cell preservation solution) comprising a trehalose group, having a pH of from 6.5 to 8.5, and having specific use "for use in preserving a mammalian cell". Note that the trehalose-containing lactated Ringer's solution and the trehalose- and dextran-containing lactated Ringer's solution used for mammalian cell preservation in Patent Document 2 are each a solution that has a pH of lower than 6.5, correspond to CSP-11 solution (Comparative Example 5 or 7) and CSP-01 solution (Comparative Example 3 or 4), respectively, in the below-described Examples, and are thus different from the present mammalian cell preservation solution.

The pH of the present mammalian cell preservation solution is permitted if the pH is within the range 6.5 to 8.5. Examples include: 6.5 to 8.4; 6.5 to 8.3; 6.5 to 8.2; 6.5 to 8.1; 6.5 to 8.0; 6.5 to 7.9; 6.5 to 7.8; 6.5 to 7.7; 6.5 to 7.6; 6.5 to 7.5; 6.5 to 7.4; 6.5 to 7.3; 6.5 to 7.2; 6.5 to 7.1; 6.5 to 7.0; 6.5 to 6.9; 6.5 to 6.8; 6.6 to 8.5; 6.7 to 8.5; 6.8 to 8.5; 6.9 to 8.5; 7.0 to 8.5; 7.1 to 8.5; 7.2 to 8.5; 7.3 to 8.5; 7.4 to 8.5; 7.5 to 8.5; 7.6 to 8.5; 7.7 to 8.5; 7.8 to 8.5; 7.9 to 8.5; 8.0 to 8.5; 8.1 to 8.5; 8.2 to 8.5; 6.6 to 8.4; 6.6 to 8.3; 6.6 to 8.2; 6.6 to 8.1; 6.6 to 8.0; 6.6 to 7.9; 6.6 to 7.8; 6.6 to 7.7; 6.6 to 7.6; 6.6 to 7.5; 6.6 to 7.4; 6.6 to 7.3; 6.6 to 7.2; 6.6 to 7.1; 6.6 to 7.0; 6.6 to 6.9; 6.7 to 8.4; 6.7 to 8.3; 6.7 to 8.2; 6.7 to 8.1; 6.7 to 8.0; 6.7 to 7.9; 6.7 to 7.8; 6.7 to 7.7; 6.7 to 7.6; 6.7 to 7.5; 6.7 to 7.4; 6.7 to 7.3; 6.7 to 7.2; 6.7 to 7.1; 6.7 to 7.0; 6.8 to 8.4; 6.8 to 8.3; 6.8 to 8.2; 6.8 to 8.1; 6.8 to 8.0; 6.8 to 7.9; 6.8 to 7.8; 6.8 to 7.7; 6.8 to 7.6; 6.8 to 7.5; 6.8 to 7.4; 6.8 to 7.3; 6.8 to 7.2; 6.8 to 7.1; 6.9 to 8.4; 6.9 to 8.3; 6.9 to 8.2; 6.9 to 8.1; 6.9 to 8.0; 6.9 to 7.9; 6.9 to 7.8; 6.9 to 7.7; 6.9 to 7.6; 6.9 to 7.5; 6.9 to 7.4; 6.9 to 7.3; 6.9 to 7.2; 7.0 to 8.4; 7.0 to 8.3; 7.0 to 8.2; 7.0 to 8.1; 7.0 to 8.0; 7.0 to 7.9; 7.0 to 7.8; 7.0 to 7.7; 7.0 to 7.6; 7.0 to 7.5; 7.0 to 7.4; 7.0 to 7.3; 7.1 to 8.4; 7.1 to 8.3; 7.1 to 8.2; 7.1 to 8.1; 7.1 to 8.0; 7.1 to 7.9; 7.1 to 7.8; 7.1 to 7.7; 7.1 to 7.6; 7.1 to 7.5; 7.1 to 7.4; 7.2 to 8.4; 7.2 to 8.3; 7.2 to 8.2; 7.2 to 8.1; 7.2 to 8.0; 7.2 to 7.9; 7.2 to 7.8; 7.2 to 7.7; 7.2 to 7.6; 7.2 to 7.5; 7.3 to 8.4; 7.3 to 8.3; 7.3 to 8.2; 7.3 to 8.1; 7.3 to 8.0; 7.3 to 7.9; 7.3 to 7.8; 7.3 to 7.7; 7.3 to 7.6; 7.4 to 8.4; 7.4 to 8.3; 7.4 to 8.2; 7.4 to 8.1; 7.4 to 8.0; 7.4 to 7.9; 7.4 to 7.8; 7.4 to 7.7; 7.5 to 8.4; 7.5 to 8.3; 7.5 to 8.2; 7.5 to 8.1; 7.5 to 8.0; 7.5 to 7.9; 7.5 to 7.8; 7.6 to 8.4; 7.6 to 8.3; 7.6 to 8.2; 7.6 to 8.1; 7.6 to 8.0; 7.6 to 7.9; 7.7 to 8.4; 7.7 to 8.3; 7.7 to 8.2; 7.7 to 8.1; 7.7 to 8.0; 7.8 to 8.4; 7.8 to 8.3; 7.8 to 8.2; 7.8 to 8.1; 7.9 to 8.4; 7.9 to 8.3; 7.9 to 8.2; 8.0 to 8.4; 8.0 to 8.3; or 8.1 to 8.3.

The present mammalian cell preservation solution may be a solution allowing for mammalian cell preservation (e.g., an isotonic solution, a hypotonic solution, a hypertonic solution). Preferable examples include an isotonic solution. As used herein, the term "isotonic solution" means a solution having substantially the same osmotic pressure as the osmotic pressure of body fluid or cell fluid. Specifically, the term means a solution having an osmotic pressure in the range of 250 to 380 mOsm/L. In addition, as used herein, the term "hypotonic solution" means a solution having an osmotic pressure lower than the osmotic pressure of body fluid or cell fluid. Specifically, the term means a solution having an osmotic pressure of less than 250 mOsm/L. It is preferable that such a hypotonic solution is a hypotonic solution so as not to lyse the cell (specifically, a solution having an osmotic pressure in the range of 100 to less than 250 mOsm/L). Further, as used herein, the term "hypertonic solution" means a solution having an osmotic pressure higher than the osmotic pressure of body fluid or cell fluid. Specifically, the term means that the osmotic pressure is more than 380 mOsm/L (preferably in the range of more than 380 mOsm/L to 1000 mOsm/L).

The above isotonic solution is not particularly limited if, for example, the salt concentration and/or the sugar concentration of the isotonic solution have been adjusted to have substantially the same osmotic pressure as of body fluid or cell fluid by using, for instance, a sodium ion, a potassium ion, and/or a calcium ion. Specific examples include saline, buffered saline (e.g., PBS, Tris Buffered Saline [TBS], HEPES Buffered Saline), Ringer's solution, lactated Ringer's solution, acetate Ringer's solution, bicarbonate Ringer's solution, 5% glucose solution, basic medium for animal cell culture (e.g., DMEM, EMEM, RPMI-1640, α-MEM, F-12, F-10, M-199), or an isotonic agent (e.g., glucose, D-sorbitol, D-mannitol, lactose, sodium chloride). Among them, lactated Ringer's solution is preferable. The isotonic solution may be commercially available or may be self-prepared. Examples of the commercially available one include Otsuka Seishoku Injection (manufactured by Otsuka Pharmaceutical Factory, Inc.) (saline solution), Ringer's solution "Otsuka" (manufactured by Otsuka Pharmaceutical Factory, Inc.) (Ringer's solution), Lactec (registered trademark) Injection (manufactured by Otsuka Pharmaceutical Factory, Inc.) (lactated Ringer's solution), Veen (registered trademark) F Inj. (manufactured by Fuso Pharmaceutical Industries, Ltd.) (acetate Ringer's solution), Otsuka sugar solution 5% (manufactured by Otsuka Pharmaceutical Factory, Inc.) (5% glucose solution), or Bicanate (registered trademark) Injection (manufactured by Otsuka Pharmaceutical Factory, Inc.) (bicarbonate Ringer's solution).

The present mammalian cell preservation solution may be produced such that the pH is adjusted to 6.5 to 8.5 by adding a pH modifier hydrogen carbonate to a trehalose group-containing solution or a solution to which a trehalose group-containing powder has been added. Examples of the above hydrogen carbonate include ammonium bicarbonate, potassium bicarbonate, sodium bicarbonate, or calcium bicarbonate. Sodium bicarbonate is preferable. In addition, pH buffering action may be absent in the present mammalian cell preservation solution, the pH of which has been adjusted using the above hydrogen carbonate.

Examples of trehalose among the above trehalose groups include, in addition to α,α-trehalose, which is a disaccharide consisting of two α-glucoses linked by a 1,1-glycosidic linkage, α,β-trehalose, which is a disaccharide consisting of α-glucose and β-glucose linked by a 1,1-glycosidic linkage, or β,β-trehalose, which is a disaccharide consisting of two β-glucoses linked by a 1,1-glycosidic linkage. Among them, α,α-trehalose is preferable. Each trehalose may be produced by a known procedure such as any of chemical synthesis, microbial production, or enzymatic production, and commercially available products may be used. Examples include commercially available α,α-trehalose (manufactured by Hayashibara Co., Ltd. or manufactured by FUJIFILM Wako Chemicals).

A trehalose derivative among the above trehalose groups is not particularly limited as long as one or more sugar units are linked to a disaccharide trehalose to yield a glycosyl trehalose group. Examples of the glycosyl trehalose group include glucosyl trehalose, maltosyl trehalose, or maltotriosyl trehalose.

Examples of a salt of trehalose or a derivative thereof among the above trehalose groups include an acid addition salt (e.g., a hydrochloride, hydrobromide, hydroiodide, phosphate, nitrate, sulfate, acetate, propionate, toluenesulfonate, succinate, oxalate, lactate, tartrate, glycolate, methanesulfonate, butyrate, valerate, citrate, fumarate, maleate, malate), a metal salt (e.g., a sodium salt, potassium salt, calcium salt), an ammonium salt, or an alkylammonium salt. Note that each salt is used in a solution form upon use, and the action is preferably the same as in the case of trehalose. Each salt compound may form a hydrate or solvate. Also, any of them may be used singly or two or more kinds thereof may be used in combination, if appropriate.

The concentration of trehalose group in the present mammalian cell preservation solution is permitted if the effect of the trehalose group on suppressing a decrease in cell viability can be exploited at the concentration. For example, the concentration in terms of trehalose is 0.1% (w/v) or higher, preferably 0.3% (w/v) or higher, more preferably 0.6% (w/v) or higher, still more preferably 1.0% (w/v) or higher, and most preferably 2.0% (w/v) or higher. In addition, from the viewpoint of avoiding a harmful effect on the cell, the concentration in terms of trehalose is, for example, 40% (w/v) or lower, preferably 20% (w/v) or lower, more preferably 15% (w/v) or lower, still more preferably 10% (w/v) or lower, and most preferably 6.0% (w/v) or lower. Thus, the concentration of trehalose group in the present mammalian cell preservation solution in terms of trehalose is, for example, in the range of 0.1 to 40% (w/v), preferably 0.3 to 20% (w/v), more preferably 0.6 to 15% (w/v), still more preferably 1.0 to 10% (w/v), and most preferably 2.0 to 6.0% (w/v).

The present mammalian cell preservation solution is used to preserve a mammalian cell for a given period under temperature conditions in which the present mammalian cell preservation solution comprising a mammalian cell is present in a liquid state. The present mammalian cell preservation solution can exert an effect of effectively suppressing a decrease in cell viability occurring when mammalian cells are preserved in liquid and/or a decrease in self-renewal potential occurring when mammalian stem cells are preserved in liquid. Further, this solution is less likely to cause a harmful effect on the life of a mammal in the case of *in vivo* administration to the mammal. Because of this, the present mammalian cell preservation solution is preferably specified by use "for use in suppressing a decrease in viability of mammalian cell", use "for use in suppressing a decrease in self-renewal potential of mammalian cell", and/or use "for use in mammalian cell transplantation".

The present mammalian cell preservation solution may be a solution comprising a trehalose group singly, a solution comprising two or more compounds selected from trehalose groups, or a solution further optionally comprising a given component in addition to the trehalose group.

As used herein, examples of the "given component" include an isotonic agent (e.g., glucose, sorbitol, mannitol, lactose, sodium chloride), a chelator (e.g., EDTA, EGTA, citric acid, salicylate), a dissolution aid, a preservative, an antioxidant, an amino acid (e.g., proline, glutamine), a polymer (e.g., polyether), a phospholipid (e.g., lysophosphatidic acid [LPA]), or a pH modifier other than a hydrogen carbonate (e.g., an alkali such as hydroxide, acetate, or carbonate; an acid such as citric acid, succinic acid, acetic acid, lactic acid, glacial acetic acid, hydrochloric acid). As used herein, the "given component" means a component that may be or is not necessarily included.

In addition, the present invention encompasses a powder preparation for preparing the present mammalian cell preservation solution comprising a trehalose group. This powder preparation optionally contains any of the above given components.

The trehalose group alone in the present mammalian cell preservation solution can elicit an effect of suppressing a decrease in viability of mammalian cell. Thus, this solution may be free of a component, except for the trehalose group, that exerts the effect of suppressing a decrease in viability of mammalian cell (e.g., a polysaccharide such as acarbose, stachyose, dextran, hydroxyethyl starch [HES], or a derivative thereof, or a salt thereof; a monosaccharide such as glucose, or a derivative thereof, or a salt thereof). However, to enhance the effect of suppressing a decrease in viability of mammalian cell, the above component, specifically a polysaccharide or a derivative thereof, or a salt thereof is preferably further optionally included. Because the effect has been demonstrated in the below-described Examples, for instance, dextran or a derivative thereof, or a salt thereof (hereinafter, they are sometimes generally referred to as a "dextran compound") may be further preferably and optionally included.

The dextran among the above dextran compounds is not particularly limited as long as the dextran is a polysaccharide $(C_6H_{10}O_5)_n$ consisting of D-glucose molecules having an $\alpha 1 \rightarrow 6$ linkage as a main chain. Examples of the dextran with a weight-average molecular weight (Mw) include dextran 40 (Mw = 40000) or dextran 70 (Mw = 70000). Each dextran may be produced by a known procedure such as any of chemical synthesis, microbial production, or enzymatic production, and commercially available products may be used. Examples include a commercially available product such as dextran 40 (manufactured by TOKYO CHEMICAL INDUSTRY CO., LTD.), dextran 70 (manufactured by TOKYO CHEMICAL INDUSTRY CO., LTD.), or low-molecular-weight Dextran L Injection (10% [w/v] dextran-containing lactated Ringer's solution) (manufactured by Otsuka Pharmaceutical Factory, Inc.).

Examples of the dextran derivative among the above dextran compounds include dextran sulfate, carboxylated dextran, or diethylaminoethyl (DEAE)-dextran.

Examples of a salt of dextran or a derivative thereof among the above dextran compounds include an acid addition salt (e.g., a hydrochloride, hydrobromide, hydroiodide, phosphate, nitrate, sulfate, acetate, propionate, toluenesulfonate, succinate, oxalate, lactate, tartrate, glycolate, methanesulfonate, butyrate, valerate, citrate, fumarate, maleate, malate), a metal salt (e.g., a sodium salt, potassium salt, calcium salt), an ammonium salt, or an alkylammonium salt. Note that each salt is used in a solution form upon use, and the action is preferably the same as in the case of dextran. Each salt compound may form a hydrate or solvate. Also, any of them may be used singly or two or more kinds thereof may be used in combination, if appropriate.

The concentration of dextran compound in the present mammalian cell preservation solution is permitted if the effect of the dextran compound on suppressing a decrease in cell viability can be exploited at the concentration. For example, the concentration in terms of dextran is 0.1% (w/v) or higher, preferably 0.3% (w/v) or higher, more preferably 0.6% (w/v) or higher, still more preferably 1.0% (w/v) or higher, still more preferably 2.0% (w/v) or higher, and most preferably 4.0% (w/v) or higher. In addition, from the viewpoint of avoiding a harmful effect on the cell, the concentration in terms of dextran is, for example, 50% (w/v) or lower, preferably 20% (w/v) or lower, more preferably 15% (w/v) or lower, still more preferably 12% (w/v) or lower, still more preferably 9.0% (w/v) or lower, and most preferably 7.0% (w/v) or lower. Thus, the concentration of dextran compound in the present mammalian cell preservation solution in terms of dextran is, for example, in the range of 0.1 to 50% (w/v), preferably 0.3 to 20% (w/v), more preferably 0.6 to 15% (w/v), still more preferably 1.0 to 12% (w/v), still more preferably 2.0 to 9.0% (w/v), and most preferably 4.0 to 7.0% (w/v).

When the present mammalian cell preservation solution comprising mammalian cells is used, as it is, for transplantation, the present mammalian cell preservation solution is preferably a solution suitable for mammalian cell transplantation. Such a solution suitable for mammalian cell transplantation is preferable free of any substance unsuitable for mammalian cell transplantation. Examples of the substance include: an *in vivo* component (e.g., serum or a serum-derived component [e.g., albumin]); or a component that suppresses a decrease in viability of mammalian cells when they are subjected to cryopreservation or lyophilization preservation, such as a cryoprotectant or lyophilization protectant (e.g., dimethyl sulfoxide [DMSO], glycerin, ethylene glycol, trimethylene glycol, dimethylacetamide, polyethylene glycol [PEG], polyvinylpyrrolidone, serum or a serum-derived component (e.g., albumin)).

A method of preserving a mammalian cell according to the present invention (hereinafter, sometimes referred to as "this preservation method") includes the step of preserving a mammalian cell for a given period in a solution comprising a trehalose group and having a pH of 6.5 to 8.5. In this preservation method, the present mammalian cell preservation solution comprising a mammalian cell is usually kept under temperature conditions in which the preservation solution is present in a liquid state. This preservation method does not include a step of preservation under temperature conditions in which the preservation solution is present in a solid state (e.g., a step of preserving a mammalian cell in a dormant state, such as a cryopreservation step or a lyophilization preservation step). In addition, the density of mammalian cells in the present mammalian cell preservation solution is, for example, in the range of $10^3$ to $10^{10}$ cells/mL.

This preservation method further optionally includes: a step of adjusting a pH to 6.5 to 8.5 by adding a hydrogen carbonate as the above pH modifier to a trehalose group-containing solution or a solution to which a trehalose group-containing powder has been added to prepare the present mammalian cell preservation solution; and/or a step of preparing a trehalose group-containing solution by adding a mammalian cell to a trehalose group-containing solution (preferably an isotonic solution) or adding a trehalose group to a mammalian cell-containing solution (preferably an isotonic solution) before the mammalian cells are preserved in the present mammalian cell preservation solution.

The given period for preserving a mammalian cell in the present invention is preferably a period allowing for an increase in the percentage of viable cells while a decrease in cell viability of mammalian cells in this preservation solution is suppressed when the present mammalian cell preservation solution comprising a mammalian cell is preserved in a liquid state. The period is, for example, 6 h or longer, 12 h or longer, 1 day (24 h) or longer, 1.5 days (36 h) or longer, 2 days (48 h) or longer, 3 days (72 h) or longer, 4 days (96 h) or longer, or 7 days (168 h) or longer. In addition, a too long mammalian cell preservation period may cause a harmful effect on the survival of cells. Thus, from the viewpoint of avoiding the harmful effect on the cell viability, the period is, for example, 21 days or shorter, 16 days or shorter, 14 days or shorter, 10 days or shorter, 7 days or shorter, or 4 days or shorter. Accordingly, examples of the above preservation period include: 6 h to 21 days; 12 h to 21 days; 1 to 21 days; 1.5 to 21 days; 2 to 21 days; 3 to 21 days; 4 to 21 days; 7 to 21 days; 6 h to 16 days; 6 h to 14 days; 6 h to 10 days; 6 h to 7 days; 6 h to 4 days; 12 h to 16 days; 12 h to 14 days; 12 h to 10 days; 12 h to 7 days; 12 h to 4 days; 1 to 16 days; 1 to 14 days; 1 to 10 days; 1 to 7 days; 1 to 4 days; 1.5 to 16 days; 1.5 to 14 days; 1.5 to 10 days; 1.5 to 7 days; 1.5 to 4 days; 2 to 16 days; 2 to 14 days; 2 to 10 days; 2 to 7 days; 2 to 4 days; 3 to 16 days; 3 to 14 days; 3 to 10 days; 3 to 7 days; 3 to 4 days; 4 to 16 days; 4 to 14 days; 4 to 10 days; 4 to 7 days; 7 to 16 days; 7 to 14 days; or 7 to 10 days. Because the effect has been demonstrated in the below-described Examples, the period may be preferably, for instance, 6 h to 14 days. Cell death of mammalian cells preserved in the present mammalian cell preservation solution may be suppressed. This can be checked using a known cell death-detectable method such as trypan blue staining, TUNEL method, Nexin method, or FLICA method.

The "temperature at which the present mammalian cell preservation solution comprising a mammalian cell is present in a liquid state" in the present invention may be a temperature at which the present mammalian cell preservation solution comprising a mammalian cell is present in a liquid but not frozen state and mammalian cells in this preservation solution can grow. The temperature is usually in the range 0 to 40°C and preferably in the range 0 to 30°C (room temperature).

The mammalian cell in the present invention is, for example, a mammalian cell administered through a blood vessel in regenerative medicine against a disease in need of mammalian cell transplantation therapy (e.g., an organ disease such as cancer, type I diabetes, or liver disease). Specific examples of the cell include a stem cell, a pancreatic island cell, a hepatocyte, or a dendritic cell. A stem cell or a hepatocyte is preferable. Each cell may be isolated by a known common procedure. For instance, a fluorescently activated cell sorter (FACS) using each antibody against each cell surface marker or an automated magnetic cell separator (autoMACS) using an antibody against the above cell surface marker as labeled with a fluorescent substance or a label such as biotin or avidin and MACS beads (magnetic beads) conjugated to an antibody against such a label may be used. Examples of the above fluorescent substance include allophycocyanin (APC), phycoerythrin (PE), FITC (fluorescein isothiocyanate), Alexa Fluor 488, Alexa Fluor 647, Alexa Fluor 700, PE-Texas Red, PE-Cy5, or PE-Cy7.

Meanwhile, the above "stem cell" means an immature cell having self-renewal potential and differentiation-proliferation potential. Depending on the differentiation potential, the stem cells include, for instance, a pluripotent stem cell, multipotent stem cell, or unipotent stem cell subpopulation. The pluripotent stem cell means a cell that can be differentiated into every tissue or cell constituting a living body whereas the cell, by itself, cannot become an organism. The multipotent stem cell means a stem cell that can be differentiated into multiple tissues and/or cells, but not into all the types. The unipotent stem cell means a stem cell that can be differentiated into certain tissues and/or cells.

Examples of the above pluripotent stem cells include embryonic stem cells (ES cells), embryonic germ cells (EG cells), or induced pluripotent stem cells (iPS cells). ES cells can be produced by culturing an inner cell mass on feeder cells or in a LIF-containing culture medium. The ES cell production protocol is described in, for example, WO96/22362, WO02/101057, US5,843,780, US6,200,806, or US6,280,718. EG cells can be produced by culturing primordial germ cells in a culture medium comprising mSCF, LIF, and bFGF (Cell, 70: 841-847, 1992). iPS cells can be produced by introducing, into a somatic cell (e.g., a fibroblast, a skin cell), reprogramming factors such as Oct3/4, Sox2, and Klf4 (optionally further including c-Myc or n-Myc) (Cell, 126: p.663-676,2006; Nature, 448: p.313-317, 2007; Nat Biotechnol, 26, p.101-106, 2008; Cell 131: p.861-872, 2007; Science, 318: p.1917-1920, 2007; Cell Stem Cells 1: p.55-70, 2007; Nat Biotechnol, 25: p.1177-1181, 2007; Nature, 448: p.318-324, 2007; Cell Stem Cells 2: p.10-12, 2008; Nature 451: p.141-146, 2008; or Science, 318: p.1917-1920, 2007). Stem cells established by culturing an early embryo produced by nuclear transfer of a somatic cell nucleus are also preferable as pluripotent stem cells (Nature, 385, 810 (1997); Science, 280, 1256 (1998); Nature Biotechnology, 17, 456 (1999); Nature, 394, 369 (1998); Nature Genetics, 22, 127 (1999); Proc. Natl. Acad. Sci. USA, 96, 14984 (1999), Rideout III and colleagues (Nature Genetics, 24, 109 (2000)).

Examples of the above multipotent stem cell include a somatic stem cell: a mesenchymal stem cell that can be differentiated into cells such as adipocytes, bone cells, chondrocytes, and fat cells; a neural stem cell that can be differentiated into cells such as neurons, astrocytes, and oligodendrocytes; a bone marrow stem cell; or a germline stem cell. The multipotent stem cell is preferably a mesenchymal stem cell. The mesenchymal stem cell means a stem cell that can be differentiated into all or some of osteoblasts, chondroblasts, and adipoblasts. The multipotent stem cells themselves may be isolated from a living body by a known procedure. For example, mesenchymal stem cells may be collected from, for instance, a bone marrow, an adipose tissue, peripheral blood, or cord blood of a mammal by a known common procedure. For example, human mesenchymal stem cells may be isolated by culturing and subculturing hematopoietic stem cells obtained after bone marrow puncture (Journal of Autoimmunity, 30 (2008), 163-171). Multipotent stem cells may be obtained by culturing the above pluripotent stem cells under suitable induction conditions.

Examples of the mammal in the present invention include a rodent (e.g., a mouse, a rat, a hamster, a guinea pig), the order *Lagomorpha* (e.g., a rabbit), the order *Ungulata* (e.g., a pig, a cow, a goat, a horse, sheep), the order *Carnivora* (e.g., a dog, a cat), or a primate (e.g., a human, a monkey, a rhesus monkey, a crab-eating macaque, a marmoset, an orangutan, a chimpanzee). Among them, a mouse, a pig, or a human is a preferable example.

Examples of the mammalian cell in the present invention include an adherent (also referred to as "adhesive") cell. As used herein the "adherent" cell means an anchorage-dependent cell that can survive, proliferate, and produce substances while attached to a scaffold. Examples of the adherent stem cell include a pluripotent stem cell, a mesenchymal stem cell, a neural stem cell, a bone marrow stem cell, or a germline stem cell. Preferable examples include a mesenchymal stem cell.

The mammalian cells (population) in the present invention may be isolated *in vivo* or may be subcultured *in vitro*. However, the mammalian cells are preferably isolated or purified. As used herein, the term "isolated or purified" means that an operation of removing components other than a component of interest has been carried out. The purity of isolated or purified mammalian cells (the percentage (e.g., the mammalian stem cell count) of cell of interest based on the total cell count) is usually 30% or higher, preferably 50% or higher, more preferably 70% or higher, and still more preferably 90% or higher (e.g., 100%).

The mammalian cells (population) preserved in the present mammalian cell preservation solution may be in a single cell state. As used herein, the term "single cell state" means that some cells do not assemble to form a mass (i.e., in a non-aggregated state). The mammalian cells in a single cell state may be prepared by subjecting *in vitro* cultured mammalian cells to, for instance, trypsin/EDTA enzymatic treatment. The percentage of mammalian cells in a single cell state as included in the mammalian cells is, for example, 70% or higher, preferably 90% or higher, more preferably 95% or higher, and still more preferably 99% or higher (e.g., 100%). The percentage of cells in a single cell state may be determined by dispersing mammalian cells in PBS, observing the cells under a microscope, and examining whether any aggregate is present or absent in a plurality of randomly selected cells (e.g., 1000 cells).

The mammalian cells (population) preserved in the present mammalian cell preservation solution may be suspended. As used herein, the term "suspended" means that mammalian cells are kept in solution without contact with an inner wall of cultureware containing a preservation solution.

Mammalian cells preserved in the present mammalian cell preservation solution may be aggregated or precipitated. In this case, the mammalian cells are preferably suspended by a well-known procedure in the art, such as pipetting or tapping prior to transplantation.

**Examples**

Hereinafter, the present invention will be further described in detail with reference to Examples. However, the technical scope of the present invention is not limited to these Examples. Note that in the following Examples, lactated Ringer's solution comprising 3% (w/v) trehalose and 5% (w/v) dextran 40 is sometimes referred to as "CSP-01 solution" for convenience; and lactated Ringer's solution comprising 3% (w/v) trehalose is sometimes referred to as "CSP-11 solution" for convenience.

I To Examine Suitable pH

1. Materials and Methods

[Mammalian Cells]

Human adipose tissue-derived mesenchymal stem cells (hAD-MSC) designated in Table 1 below were used in the following Experiments 1 to 4.

[Table 1]

| | |
|---|---|
| Doner's age, sex, source | 38 years old, female, fat |
| Number of doners | 1 |
| Lot No. | 0000421627 |
| Passage number when purchased | Passage 1 |
| Preservation method | Preserved in liquid nitrogen |
| Manufacturer | Lonza Walkersville, Inc. |
| Provider | Lonza Japan, Inc. |

[CSP-01 solution]

CSP-01 solution was prepared by adding, to lactated Ringer's solution (Lactec Injection; manufactured by Otsuka Pharmaceutical Factory, Inc.), α,α-trehalose (manufactured by Hayashibara Co., Ltd. or manufactured by FUJIFILM Wako Chemicals) and low-molecular-weight Dextran L Injection (10% [w/v] dextran-containing lactated Ringer's solution) (manufactured by Otsuka Pharmaceutical Factory, Inc.) so that the trehalose or the dextran had a final concentration of 3% (w/v) or 5% (w/v), respectively (see Patent Document 2).

[CSP-11 solution]

CSP-11 solution was prepared by adding, to lactated Ringer's solution (Lactec Injection; manufactured by Otsuka Pharmaceutical Factory, Inc.), α,α-trehalose (manufactured by Hayashibara Co., Ltd. or manufactured by FUJIFILM Wako Chemicals) so that the trehalose had a final concentration of 3% (w/v) (see Patent Document 2). Table 2 below shows the composition of CSP-01 solution or CSP-11 solution.

[Table 2]

|  | CSP-01 solution | CSP-11 solution |
|---|---|---|
| Dextran 40 (w/v)% | 5 | - |
| Trehalose (w/v)% | 3 | 3 |
| Sodium chloride (w/v)% | 0.6 | 0.6 |
| Calcium chloride (w/v)% | 0.02 | 0.02 |
| Potassium chloride (w/v)% | 0.03 | 0.03 |
| Sodium L-lactate (w/v)% | 0.31 | 0.31 |

[To Culture hAD-MSC]

hAD-MSC were cultured in accordance with a defined protocol. Specifically, hAD-MSC were placed in a 75-$cm^2$ flask with ADSC-BM (Adipose Derived Stem Cell Basal Medium) (PT-3273, manufactured by Lonza Walkersville, Inc.) containing a human adipose-derived stem cell supplemental factor set (PT-4503, manufactured by Lonza Walkersville, Inc.) (hereinafter, simply referred to as "culture medium"), and then subcultured in a $CO_2$ incubator (under conditions at 37°C). Meanwhile, the culture medium was changed every three days.

[To Prepare hAD-MSC-Containing solution]

The hAD-MSC-containing solution was prepared in accordance with the following procedures [1] to [10].

[1] Warm a personal incubator at 37 ± 2°C.
[2] Take out a 75-$cm^2$ flask having cultured hAD-MSC from a $CO_2$ incubator.
[3] Observe the cell conditions under an inverted microscope and use about 80%-confluent ones.
[4] Aspirate the culture medium and add 8 mL of PBS (-) to each 75-$cm^2$ flask.
[5] Aspirate the PBS (-) and then add 4 mL of trypsin/EDTA (CC-5012, manufactured by Lonza Walkersville, Inc.) to each flask, and incubate the cells for 5 min in the personal incubator under conditions at 37 ± 2°C.
[6] Gently rock the cells while observed under an inverted microscope until about 90% has detached.
[7] Add 8 mL of trypsin neutralizing solution (TNS; CC-5002, manufactured by Lonza Walkersville, Inc.) to stop the trypsin reaction, detach the cells by pipetting, and transfer the cells to a 50-mL conical tube.
[8] Centrifuge the tube (at 210 × g for 5 min at 20°C) to remove the supernatant, add a certain volume (1.5 mL per 75-$cm^2$ flask) of PBS (-), and suspend the cells.
[9] Take and mix a fraction (20 pL) of cell suspension with 20 μL of trypan blue staining solution (manufactured by Gibco, Inc.), and measure the total cell count (viable cell count and dead cell count) by using a OneCell counter (a cell-counter; manufactured by Bio-Medical Science Co., Ltd.). Note that 4 corner areas out of 9 areas on the OneCell counter were used to count the cells and the same applied to the following counting.
[10] Add PBS (-) so as to set the total cell count to $5.0 \times 10^5$ cells/mL, and cool the cells on ice to prepare a mammalian cell-containing solution.

[To Measure Cell Viability and Viable Cell Recovery Rate]

The cell viability and the viable cell recovery rate when mammalian cells were preserved in a test solution were measured in accordance with the following procedures [1] to [4].

[1] Dispense 1 mL of the prepared mammalian cell-containing solution into each 15-mL clarified polypropylene conical tube by using a Finnpipette (100-1000 µL), and centrifuge (at 210 × g for 5 min at 25°C) and then cool the solution on ice.

[2] Remove the supernatant, suspend the cells in 1 mL of each test solution by using a Finnpipette (100-1000 µL), and put each aliquot (20 pL) into a 1.5-mL microfuge tube already containing 20 µL of trypan blue staining solution (manufactured by Gibco, Inc.). Place, onto a OneCell counter, a cell suspension mixed with the trypan blue staining solution, and measure the total cell count and the trypan blue-positive cell (dead cell) count by using a light microscope (ECLIPSE TS100, manufactured by Nikon, Inc.) to calculate the cell viability immediately after the start of preservation.

[3] Preserve the rest cell suspension while allowed to stand for each preservation period in a refrigerated chemical case (set to 5°C).

[4] Insert, at the timepoint when each preservation period has passed, the tip end into the tube with the cell-containing solution to reach the position visually about 5 mm from the bottom; collect, into a 1.5-mL microfuge tube, each aliquot (20 pL) with the cells suspended by gentle mixing (pipetting 5 times using a solution volume of 500 pL); mix the solution with 20 µL of trypan blue staining solution (manufactured by Gibco, Inc.); then place the mixture onto a OneCell counter; and measure the total cell count and the trypan blue-positive cell (dead cell) count by using a light microscope (ECLIPSE TS100, manufactured by Nikon, Inc.) to calculate the cell viability and the viable cell recovery rate for each preservation period. Note that the cell viability was calculated by using the formula: "(Total viable cell count/total cell count) × 100 = ([Total cell count - Dead cell count]/Total cell count) × 100 = Cell viability (%)". In addition, the viable cell recovery rate was calculated by using the formula: "(Total viable cell count for each preservation period/Total viable cell count immediately after the start of preservation) × 100 = ([Total cell count for each preservation period - Dead cell count for each preservation period]/[Total cell count immediately after the start of preservation - Dead cell count immediately after the start of preservation]) × 100 = Viable cell recovery rate (%)".

[CFU Assay]

The colony-forming ability when mammalian mesenchymal stem cells were preserved in a test solution was measured in accordance with the following procedures [1] to [4].

[1] Dispense 1 mL of the prepared mammalian cell-containing solution into each 15-mL clarified polypropylene conical tube by using a Finnpipette (100-1000 µL), and centrifuge (at 210 × g for 5 min at 25°C) and then cool the solution on ice.

[2] Remove the supernatant and suspend the cells in 1 mL of each test solution by using a Finnpipette (100-1000 µL); then plate its portion (20 pL) at 15 cells/cm$^2$ on a 60-mm dish (with an area of 21 cm$^2$): and measure the number of colonies formed after about 8 days to calculate the colony-forming unit (CFU), that is, the ratio of the colony count to the plated cell count, immediately after the start of preservation.

[3] Preserve the rest cell suspension while allowed to stand for each preservation period in a refrigerated chemical case (set to 5°C).

[4] Insert, at the timepoint when each preservation period has passed, the tip end into the tube with the cell-containing solution to reach the position visually about 5 mm from the bottom; seed the cells at 15 cells/cm$^2$ on a 60-mm dish (with an area of 21 cm$^2$) with the cells suspended by gentle mixing (pipetting 5 times using a solution volume of 500 pL); and measure the number of colonies formed after about 8 days to calculate the CFU immediately after the start of preservation.

2. Results

[Experiment 1]

The cell viability (see Table 4 and Figure 1A) and the viable cell recovery rate (see Table 5 and Figure 1B) when hAD-MSC were preserved for each preservation period (1 day, 2 days, 4 days, 7 days, or 14 days) in 6 different test solutions designated in Table 3 below were measured in accordance with the protocol described in the above section [To Measure Cell Viability and Viable Cell Recovery Rate].

[Table 3]

|  | Test solution | pH modifier (addition amount [g/L]) |
|---|---|---|
| Comparative Example 1 | Lactated Ringer's | - |
| Comparative Example 2 | CSP-01 solution (pH 5.65) | Hydrochloric acid (0.0350) |

(continued)

| | Test solution | pH modifier (addition amount [g/L]) |
|---|---|---|
| Comparative Example 3 | CSP-01 solution (pH6.15) | - |
| Example 1 | CSP-01 solution (pH6.60) | Sodium bicarbonate (0.0125) |
| Example 2 | CSP-01 solution (pH6.95) | Sodium bicarbonate (0.0250) |
| Example 3 | CSP-01 solution (pH7.29) | Sodium bicarbonate (0.0350) |

The results revealed that in Comparative Example 1 (lactated Ringer's solution) or Comparative Example 2 (CSP-01 solution [pH 5.65]), the cell viability at day 2 after the start of cell preservation was decreased to about 65% in each case and the cell viability at day 4 was decreased to about 30% or less in each case (see Table 4 and Figure 1A). By contrast, in Examples 1 to 3 (CSP-01 solution s [pH 6.60 to 7.29]), the cell viability at day 2 after the start of cell preservation exceeded 96% in each case and the cell viability at day 4 exceeded 70% in each case (see Table 4 and Figure 1A). In addition, the cell viability at day 4 or later after the start of cell preservation was compared among Examples 1 to 3. Here, the cell viability was higher in Example 2 (CSP-01 solution [pH 6.95]) than in Example 1 (CSP-01 solution [pH 6.60]); and the cell viability was higher in Example 3 (CSP-01 solution [pH 7.29]) than in Example 2 (CSP-01 solution [pH 6.95]) (see Table 4 and Figure 1A). Also, a similar trend was recognized for the viable cell recovery rate (see Table 5 and Figure 1B).

The results have demonstrated as follows: when the pH of CSP-01 solution, that is, lactated Ringer's solution comprising trehalose and dextran is adjusted to about 6.6 or higher (preferably at or near about 7.29) and mammalian cells are preserved in the solution, cell death can be effectively suppressed and the percentage of viable cells can be higher than in the case where mammalian cells are preserved in lactated Ringer's solution free of trehalose or dextran, or lactated Ringer's solution comprising trehalose and dextran and having a pH of 6.15 or lower.

[Table 4]

| | Immediately after start of preservation | Preservation period | | | | |
|---|---|---|---|---|---|---|
| | | Day 1 | Day 2 | Day 4 | Day 7 | Day 14 |
| Comparative Example 1 | 98.8±1.3 | 89.3±2.0 | 65.7±5.6 | 22.7±15.9 | 10.1±3.2 | 2.0±2.1 |
| Comparative Example 2 | 99.6±0.6 | 93.5±2.0 | 66.4±7.6 | 25.1±9.0 | 23.5±3.7 | 10.4±4.3 |
| Comparative Example 3 | 99.2±0.7 | 98.6±0.5 | 96.7±1.8 | 67.8±8.9 | 43.7±4.1 | 18.7±6.8 |
| Example 1 | 99.3±1.3 | 99.6±0.6 | 97.8±1.7 | 73.9±4.0 | 59.1±3.6 | 21.8±5.5 |
| Example 2 | 99.2±0.7 | 99.2±0.7 | 97.4±0.5 | 86.8±0.3 | 61.9±2.7 | 37.8±14.0 |
| Example 3 | 99.2±0.7 | 97.9±2.2 | 96.2±2.5 | 92.5±1.4 | 66.3±0.3 | 39.3±4.7 |

The numbers in the Table each represent the cell viability (mean ± standard deviation [SD]; n = 3).

[Table 5]

| | Immediately after start of preservation | Preservation period | | | | |
|---|---|---|---|---|---|---|
| | | Day 1 | Day 2 | Day 4 | Day 7 | Day 14 |
| Comparative Example 1 | 100±0.0 | 95.8±4.8 | 68.7±12.2 | 25.9±17.3 | 9.6±1.9 | 1.60±1.8 |
| Comparative Example 2 | 100±0.0 | 95.8±7.2 | 74.2±13.3 | 27.0±9.9 | 24.0±3.5 | 12.6±6.2 |
| Comparative Example 3 | 100±0.0 | 110±11.9 | 105±17.9 | 74.2±12.7 | 48.0±6.8 | 22.4±10.0 |
| Example 1 | 100±0.0 | 106±6.6 | 99.6±4.6 | 79.4±7.6 | 64.5±6.4 | 23.7±6.6 |
| Example 2 | 100±0.0 | 105±10.8 | 105±4.3 | 102±6.0 | 67.1±7.3 | 41.8±14.3 |
| Example 3 | 100±0.0 | 110±16.2 | 107±7.3 | 107±19.1 | 81.5±2.6 | 42.8±9.6 |

The numbers in the Table each represent the viable cell recovery rate (mean ± standard deviation [SD]; n = 3).

[Experiment 2]

The cell viability (see Table 7) and the viable cell recovery rate (see Table 8) when hAD-MSC were preserved for each preservation period (6 h, 24 h, 48 h, or 96 h) in 4 different test solutions designated in Table 6 below were measured in accordance with the protocol described in the above section [To Measure Cell Viability and Viable Cell Recovery Rate].

[Table 6]

|  | Test solution | pH modifier (addition amount [g/L]) |
|---|---|---|
| Example 4 | CSP-01 solution (pH7.29) | Sodium bicarbonate (0.0350) |
| Comparative Example 4 | CSP-01 solution (pH6.16) | - |
| Example 5 | CSP-11 solution (pH7.04) | Sodium bicarbonate (0.0150) |
| Comparative Example 5 | CSP-11 solution (pH6.35) | - |

The results revealed that in Comparative Example 4 (CSP-01 solution [pH 6.16]), the cell viability at 48 h (day 2) after the start of cell preservation was decreased to about 50% and the cell viability at 96 h (day 4) was decreased to 37% (see Table 7). By contrast, in Example 4 (CSP-01 solution [pH 7.29]), the cell viability at 48 h (day 2) after the start of cell preservation exceeded 90% and the cell viability at 96 h (day 4) exceeded 70% (see Table 7). In addition, a similar trend was recognized for the viable cell recovery rate (see Table 8).

Meanwhile, in Comparative Example 5 (CSP-11 solution [pH 6.35]), the cell viability at 48 h (day 2) after the start of cell preservation was decreased to about 45% and the cell viability at 96 h (day 4) was decreased to about 15% (see Table 7). By contrast, in Example 5 (CSP-11 solution [pH 7.04]), the cell viability at 48 h (day 2) after the start of cell preservation exceeded 75% and the cell viability at 96 h (day 4) exceeded 45% (see Table 7). Also, a similar trend was recognized for the viable cell recovery rate (see Table 8).

The results have demonstrated as follows: the above [Experiment 1] results are supported; and when the pH of CSP-11 solution, that is, lactated Ringer's solution comprising trehalose (free of dextran) is also adjusted to about 7.04 or higher and mammalian cells are preserved in the solution, cell death can be effectively suppressed and the percentage of viable cells can be higher than in the case where mammalian cells are preserved in lactated Ringer's solution comprising trehalose and having a pH of 6.35 or lower.

[Table 7]

|  | Immediately after start of preservation | Preservation period | | | |
|---|---|---|---|---|---|
|  |  | 6 h | 24 h | 48 h | 96 h |
| Example 4 | 98.4±2.0 | 98.6±1.2 | 97.6±2.4* | 92.3±4.1*** | 72.5±3.4*** |
| Comparative Example 4 | 98.6±1.8 | 98.9±1.5 | 92.3±2.8 | 49.7±5.6 | 37.0±7.4 |
| Example 5 | 98.2±3.1 | 98.3±0.8 | 94.9±2.4 | 76.0±12.9* | 45.6±8.6*** |
| Comparative Example 5 | 98.7±1.0 | 97.5±1.2 | 88.5±8.4 | 45.1±14.8 | 15.8±4.1 |

The numbers in the Table each represent the cell viability (mean ± standard deviation [SD]; n = 4). The "*" and "***" in Example 4 indicate a statistically significant difference ($p < 0.05$ and $p < 0.001$, respectively) from Comparative Example 4 having the same preservation period. In addition, the "*" and "***" in Example 5 indicate a statistically significant difference ($p < 0.05$ and $p < 0.001$, respectively) from Comparative Example 5 having the same preservation period.

[Table 8]

|  | Immediately after start of preservation | Preservation period | | | |
|---|---|---|---|---|---|
|  |  | 6 h | 24 h | 48 h | 96 h |
| Example 4 | 100±0.0 | 95.1±7.3 | 102±4.3* | 104±7.7*** | 83.5±5.3*** |

(continued)

| | Immediately after start of preservation | Preservation period | | | |
|---|---|---|---|---|---|
| | | 6 h | 24 h | 48 h | 96 h |
| Comparative Example 4 | 100±0.0 | 89.5±4.7 | 91.9±4.9 | 52.2±6.9 | 40.7±8.9 |
| Example 5 | 100±0.0 | 97.3±7.0 | 93.4±3.5 | 76.2±8.1* | 46.6±8.1*** |
| Comparative Example 5 | 100±0.0 | 103±8.8 | 96.2±12.0 | 50.7±16.9 | 17.3±4.7 |

The numbers in the Table each represent the viable cell recovery rate (mean ± standard deviation [SD]; n = 4). The "*" and "***" in Example 4 indicate a statistically significant difference (p < 0.05 and p < 0.001, respectively) from Comparative Example 4 having the same preservation period. In addition, the "*" and "***" in Example 5 indicate a statistically significant difference (p < 0.05 and p < 0.001, respectively) from Comparative Example 5 having the same preservation period.

[Experiment 3]

The cell viability (see Tables 10 and 12) and the viable cell recovery rate (see Tables 11 and 13) when hAD-MSC were preserved for each preservation period (24 h, 48 h, 96 h, or 168 h) in 11 different test solutions designated in Table 9 below were measured in accordance with the protocol described in the above section [To Measure Cell Viability and Viable Cell Recovery Rate].

[Table 9]

| | Test solution | pH modifier (addition amount [g/L]) |
|---|---|---|
| Comparative Example 6 | Lactated Ringer's | - |
| Example 6 | CSP-01 solution (pH6.69) | Sodium bicarbonate (0.0125) |
| Example 7 | CSP-01 solution (pH7.03) | Sodium bicarbonate (0.0250) |
| Example 8 | CSP-01 solution (pH7.35) | Sodium bicarbonate (0.0350) |
| Example 9 | CSP-01 solution (pH7.68) | Sodium bicarbonate (0.100) |
| Example 10 | CSP-01 solution (pH8.02) | Sodium bicarbonate (0.300) |
| Comparative Example 7 | CSP-11 solution (pH6.44) | - |
| Example 11 | CSP-11 solution (pH7.04) | Sodium bicarbonate (0.0100) |
| Example 12 | CSP-11 solution (pH7.16) | Sodium bicarbonate (0.0150) |
| Example 13 | CSP-11 solution (pH7.69) | Sodium bicarbonate (0.0500) |
| Example 14 | CSP-11 solution (pH8.00) | Sodium bicarbonate (0.100) |

The results revealed that the cell viability and the viable cell recovery rate were higher for any of the preservation periods in Examples 6 to 10 (CSP-01 solutions [pH 6.69 to 8.02]) than in Comparative Example 6 (lactated Ringer's solution). In Example 8 (CSP-01 solution [pH 7.35]) and Example 9 (CSP-01 solution [pH 7.68]), in particular, the cell viability and the viable cell recovery rate tended to be the highest (see Tables 10 and 11 and Figure 2).

The results have demonstrated as follows: the above [Experiment 1] and [Experiment 2] results are supported; and when the pH of CSP-01 solution, that is, lactated Ringer's solution comprising trehalose and dextran is adjusted to 6.69 or higher and mammalian cells are preserved in the solution, cell death can be effectively suppressed and the percentage of viable cells can be higher than in the case where mammalian cells are preserved in lactated Ringer's solution.

Further, the cell viability and the viable cell recovery rate were higher for any of the preservation periods in Examples 11 to 14 (CSP-11 solutions [pH 7.04 to 8.00]) than in Comparative Example 6 (lactated Ringer's solution) or Comparative Example 7 (CSP-11 solution [pH 6.44]). In Examples 12 to 14 (CSP-11 solutions [pH 7.16 to 8.00]), in particular, the cell viability and the viable cell recovery rate tended to be the highest (see Tables 12 and 13 and Figure 3).

The results have demonstrated as follows: the above [Experiment 2] results are supported; and when the pH of CSP-11 solution, that is, lactated Ringer's solution comprising trehalose (free of dextran) is adjusted to 7.04 or higher and mammalian cells are preserved in the solution, cell death can be effectively suppressed and the percentage of viable cells can be higher than in the case where mammalian cells are preserved in lactated Ringer's solution free of trehalose or lactated Ringer's solution comprising trehalose and having a pH of 6.44 or lower.

[Table 10]

| | after start of preservation | Immediately | Preservation period | | |
| | | 24 h | 48 h | 96 h | 168 h |
|---|---|---|---|---|---|
| Comparative Example 6 | 98.0±0.1 | 83.9±3.9 | 63.5±8.4 | 35.9±7.0 | 13.3±9.3 |
| Example 6 | 98.5±0.8 | 89.5±3.5 | 79.0±2.3 | 59.4±3.6 | 44.0±6.3 |
| Example 7 | 97.4±0.7 | 93.7±1.7 | 81.6±3.5 | 64.1±1.1 | 52.3±6.7 |
| Example 8 | 99.0±0.1 | 97.3±0.5 | 90.6±1.9 | 83.8±1.9 | 56.9±5.0 |
| Example 9 | 98.2±0.6 | 94.2±2.6 | 89.2±4.8 | 79.9±3.1 | 60.9±8.1 |
| Example 10 | 98.3±0.5 | 89.8±5.5 | 83.3±7.9 | 77.7±5.9 | 51.4±8.2 |

The numbers in the Table each represent the cell viability (mean ± standard deviation [SD]; n = 3).

[Table 11]

| | Immediately after start of preservation | Preservation period | | | |
| | | 24 h | 48 h | 96 h | 168 h |
|---|---|---|---|---|---|
| Comparative Example 6 | 100±0.0 | 79.4±12.0 | 57.6±8.8 | 34.7±6.3 | 7.0±5.9 |
| Example 6 | 100±0.0 | 101±10.5 | 82.7±11.8 | 57.8±13.6 | 48.6±9.6 |
| Example 7 | 100±0.0 | 105±11.4 | 99.8±19.9 | 72.4±3.5 | 59.7±6.7 |
| Example 8 | 100±0.0 | 100±16.1 | 93.1±15.3 | 88.6±4.9 | 59.2±4.5 |
| Example 9 | 100±0.0 | 103±9.8 | 89.2±7.5 | 88.9±6.5 | 58.1±11.1 |
| Example 10 | 100±0.0 | 88.1±8.9 | 75.3±5.8 | 79.0±16.1 | 41.2±6.7 |

The numbers in the Table each represent the viable cell recovery rate (mean ± standard deviation [SD]; n = 3).

[Table 12]

| | Immediately after start of preservation | Preservation period | | | |
| | | 24 h | 48 h | 96 h | 168 h |
|---|---|---|---|---|---|
| Comparative Example 6 | 97.6±0.5 | 85.9±4.2 | 55.8±6.6 | 33.8±1.8 | 9.3±5.3 |
| Comparative Example 7 | 98.3±0.6 | 86.3±1.7 | 62.6±5.6 | 41.5±6.4 | 16.4±2.5 |
| Example 11 | 98.6±1.5 | 93.1±3.3 | 69.8±5.0 | 44.2±8.3 | 27.5±4.3 |
| Example 12 | 97.7±1.2 | 93.3±0.3 | 76.3±3.8 | 47.5±11.7 | 37.0±2.9 |
| Example 13 | 97.8±1.1 | 89.4±3.2 | 80.4±3.4 | 66.0±5.9 | 29.7±4.1 |
| Example 14 | 98.6±1.6 | 92.1±3.7 | 80.6±8.7 | 58.0±5.5 | 45.3±0.8 |

The numbers in the Table each represent the cell viability (mean ± standard deviation [SD]; n = 3).

[Table 13]

| | Immediately after start of preservation | Preservation period | | | |
|---|---|---|---|---|---|
| | | 24 h | 48 h | 96 h | 168 h |
| Comparative Example 6 | 100±0.0 | 82.4±12.9 | 53.8±12.4 | 32.5±4.2 | 6.9±5.1 |
| Comparative Example 7 | 100±0.0 | 92.9±13.9 | 65.4±14.2 | 36.9±6.2 | 12.2±2.3 |
| Example 11 | 100±0.0 | 98.6±13.2 | 82.3±17.4 | 46.3±11.5 | 24.8±2.2 |
| Example 12 | 100±0.0 | 89.1±13.3 | 74.2±5.8 | 43.5±12.8 | 30.1±8.4 |
| Example 13 | 100±0.0 | 89.1±7.2 | 82.7±11.0 | 63.3±4.6 | 23.5±6.6 |
| Example 14 | 100±0.0 | 82.8±2.6 | 80.0±13.0 | 51.3±9.0 | 32.0±6.4 |

The numbers in the Table each represent the viable cell recovery rate (mean ± standard deviation [SD]; n = 3).

[Experiment 4]

The colony-forming ability (see Table 15) when hAD-MSC were preserved for each preservation period (6 h, 24 h, or 48 h) in 4 different test solutions designated in Table 14 below were measured in accordance with the protocol described in the above section [CFU Assay].

[Table 14]

| | Test solution | pH modifier (addition amount [g/L]) |
|---|---|---|
| Example 15 | CSP-01 solution (pH7.29) | Sodium bicarbonate (0.0350) |
| Comparative Example 8 | CSP-01 solution (pH6.16) | - |
| Example 16 | CSP-11 solution (pH7.04) | Sodium bicarbonate (0.0150) |
| Comparative Example 9 | CSP-11 solution (pH6.35) | - |

The results revealed that in Comparative Example 8 (CSP-01 solution [pH 6.16]), as the preservation period extended, the colony-forming ability of hAD-MSC decreased. By contrast, in Example 15 (CSP-01 solution [pH 7.29]), the decrease in the colony-forming ability was suppressed (see Table 15).

The results have demonstrated as follows: when the pH of CSP-01 solution, that is, lactated Ringer's solution comprising trehalose and dextran is adjusted to approximately 7.29 and mammalian mesenchymal stem cells are preserved in the solution, the decrease in the colony-forming ability was suppressed more than in the case where mammalian mesenchymal stem cells are preserved in lactated Ringer's solution comprising trehalose and dextran and having a pH at or near 6.16.

Further, in Comparative Example 9 (CSP-11 solution [pH 6.35]), as the preservation period extended, the colony-forming ability of hAD-MSC decreased. By contrast, in Example 16 (CSP-11 solution [pH 7.04]), the decrease in the colony-forming ability was suppressed (see Table 15).

The results have demonstrated as follows: when the pH of CSP-11 solution, that is, lactated Ringer's solution comprising trehalose (free of dextran) is adjusted to approximately 7.04 and mammalian stem cells are preserved in the solution, the decrease in the colony-forming ability (i.e., self-renewal potential) was suppressed more than in the case where mammalian stem cells are preserved in lactated Ringer's solution comprising trehalose and having a pH at or near 6.35.

[Table 15]

| | Immediately after start of preservation | Preservation period | | |
|---|---|---|---|---|
| | | 6 h | 24 h | 48 h |
| Example 15 | 21.3±1.9 | 21.9±3.2 | 22.0±2.7** | 15.2±3.6*** |
| Comparative Example 8 | 21.3±1.9 | 20.8±1.4 | 14.9±2.5 | 1.70±1.4 |
| Example 16 | 21.6±2.5 | 21.0±2.0 | 16.3±5.4 | 10.0±7.1* |
| Comparative Example 9 | 21.6±2.5 | 22.7±1.6 | 14.5±3.4 | 1.10±0.8 |

The numbers in the Table each represent the ratio of the number of colonies to the number of cells seeded (mean $\pm$ standard deviation [SD]; n = 4). The "**" and "***" in Example 15 indicate a statistically significant difference (p < 0.01 and p < 0.001, respectively) from Comparative Example 8 having the same preservation period. In addition, the "*" in Example 16 indicates a statistically significant difference (p < 0.05) from Comparative Example 9 having the same preservation period.

II To Examine pH Modifier

1. Materials and Methods

[Mammalian Cells]

Human bone marrow-derived mesenchymal stem cells (hBM-MSC) designated in Table 16 below were used in the following Experiments.

[Table 16]

| Test solution | Cell viability (mean $\pm$ SD) | |
|---|---|---|
| | Before preservation | Day 3 after preservation |
| CSP-01 (5.6 unadjusted) | | 79.8$\pm$7.6 |
| CSP-01 (7.3NaOH) | 98.5$\pm$0.4 | 62.7$\pm$4.3 |
| CSP-01 (7.2NaHCO$_3$) | | 87.9$\pm$1.7 |

[To Prepare Test solution]

CSP-01 solution was prepared like in Example 1, and the CSP-01 solution (5.6 unadjusted) was obtained by adding, to lactated Ringer's solution (Lactec Injection; manufactured by Otsuka Pharmaceutical Factory, Inc.), $\alpha,\alpha$-trehalose (manufactured by Hayashibara Co., Ltd. or manufactured by FUJIFILM Wako Chemicals) and low-molecular-weight Dextran L Injection (10% [w/v] dextran-containing lactated Ringer's solution) (manufactured by Otsuka Pharmaceutical Factory, Inc.) so that the trehalose or the dextran had a final concentration of 3% (w/v) or 5% (w/v), respectively. CSP-01 solution (7.3 NaOH) was obtained by adding 0.5 mol/L sodium hydroxide solution to have a pH of 7.300. A sodium bicarbonate solution was added as a pH modifier to prepare CSP-01 solution (7.2 NaHCO$_3$). The CSP-01 solution (7.2 NaHCO$_3$) had a pH of 7.158. Note that the pH of each test solution was a value measured at room temperature (21.2 to 24.0°C).

[To Prepare hBM-MSC-Containing Lactated Ringer's Solution]

The hBM-MSC-containing lactated Ringer's solution was prepared in accordance with the following procedures [1] to [8].

[1] Add $4 \times 10^5$ hBM-MSC to a 75-cm$^2$ flask and culture the cells in an incubator at 37°C and 5% $CO_2$ in the presence of a culture medium kit (manufactured by Lonza, Inc.) specialized for human mesenchymal stem cells (hereinafter, referred to as "MSC culture medium"); observe the cell conditions under a microscope; and perform culturing until the cells have become about 80% confluent.
[2] Remove the MSC culture medium and rinse the hBM-MSC with 10 mL of PBS (-).
[3] Remove the PBS (-), add 4 mL of trypsin-EDTA (CC-3232, manufactured by Lonza, Inc.), leave the cells at room temperature for 5 min.
[4] Gently rock the cells while observed under a microscope until about 90% hBM-MSC have detached.
[5] Add 5 mL of MSC culture medium to stop the trypsin reaction, and collect and transfer, by pipetting, the hBM-MSC to a 50-mL centrifuge tube.
[6] Perform centrifugation at $600 \times$ g for 5 min at room temperature.
[7] Remove the supernatant (MSC culture medium), add 9 mL of lactated Ringer's solution, and suspend the precipitates (hBM-MSC).
[8] Measure the cell count, adjust the count at $5 \times 10^5$ cells/mL by using lactated Ringer's solution, and prepare hBM-MSC-containing lactated Ringer's solution.

[Method of Preserving hBM-MSC in Various Test Isotonic Solutions]

The hBM-MSC were preserved in various test isotonic solutions in accordance with the following procedures [1] to [2].

[1] Dispense 0.5 mL of the prepared hBM-MSC-containing lactated Ringer's solution to each tube, and centrifuge each tube (at 600 × g for 5 min).
[2] Remove the supernatant (lactated Ringer's solution), suspend the precipitates (hBM-MSC) in 0.5 mL of each test isotonic solution, and preserve the suspension at 5°C for 3 days.

[To Analyze Cell Viability of hBM-MSC by Trypan Blue Staining]

Trypan blue staining was used to analyze the cell viability of hBM-MSC in accordance with the following procedures [1] to [2] .

[1] Sample 20 μL of each test isotonic solution comprising hBM-MSC after 3-day preservation at 5°C, transfer the sample to a tube, and then mix the sample with 20 μL of trypan blue staining solution (manufactured by Gibco, Inc.). Note that as a control, 20 μL of each lactated Ringer's solution comprising hBM-MSC before suspended in each test isotonic solution (before preservation at 5°C) was sampled, transferred to a tube, and then mixed with 20 μL of trypan blue staining solution.
[2] Measure the total cell count and the trypan blue-positive cell (dead cell) count by using a OneCell counter (manufactured by Bio-Medical Science Co., Ltd.) under a microscope to calculate the cell viability, that is, the ratio of the trypan blue-negative cells to the total cell count. The cell viability (%) was calculated using the following Equation 1:

[Equation 1]

$$\text{Cell viability (\%) = (Total cell count - Dead cell count)/Total cell count} \times 100.$$

2. Results

[Experiment]

The cell viability (see Figure 4) when hBM-MSC were preserved at 5°C for 3 days in 3 different tests designated in Table 17 below was measured in accordance with the protocol described in the above section [To Measure Cell viability]. Table 18 and Figure 4 show the results.

[Table 17]

| Doner's age, sex, source | 31 years old, male, bone marrow |
|---|---|
| Number of doners | 1 |
| Lot No. | 0000527428 |
| Passage number when purchased | Passage 2 |
| Preservation method | Preserved in solution nitrogen |
| Manufacturer | Lonza Walkersville, Inc. |
| Provider | KATAYAMA CHEMICAL INDUSTRIES |

[Table 18]

| Group (n = 4) | pH (measured value) | Temperature(°C) |
|---|---|---|
| CSP-01 (5.6 unadjusted) | 5.599 | 23.7 |
| CSP-01 (7.3NaOH) | 7.300 | 24.0 |

(continued)

| Group (n = 4) | pH (measured value) | Temperature(°C) |
|---|---|---|
| CSP-01 (7.2NaHCO$_3$) | 7.158 | 21.2 |

The results revealed that hBM-MSC were preserved at 5°C for 3 days; and the cell viability was significantly lower in the case of preservation in CSP-01 (7.3 NaOH) than in the case of preservation in CSP-01 (5.6 unadjusted). However, it was demonstrated that the cell viability when the cells were preserved in CSP-01 (7.2 NaHCO$_3$) was marginally higher than in the case of preservation in CSP-01 (5.6 unadjusted) or CSP-01 (7.3 NaOH).

These results have demonstrated that when the pH of CSP-01 has been adjusted to 7.2 to 7.3, the pH modifier affects the cell viability; and use of sodium bicarbonate makes it possible to maintain the cell viability higher than use of sodium hydroxide.

## Industrial Applicability

According to the present invention, addition of a trehalose group to a liquid makes it possible to effectively suppress a decrease in cell viability occurring when mammalian cells are preserved in liquid and/or a decrease in self-renewal potential occurring when mammalian stem cells are preserved in liquid. Further, the trehalose group is a disaccharide that is less likely to cause a harmful effect on the life of a mammal in the case of *in vivo* administration to the mammal. This makes it possible to *in vivo* administer to the mammal, without replacement by a fresh solution for transplantation, the mammalian cells as they are after they are preserved in the present mammalian cell preservation solution.

## Claims

1. A mammalian cell preservation solution comprising trehalose or a derivative thereof, or a salt thereof and a hydrogen carbonate as a pH modifier, and having a pH of 6.5 to 8.5.

2. The preservation solution according to claim 1, wherein the hydrogen carbonate is sodium bicarbonate.

3. The preservation solution according to claim 1 or 2, further comprising a polysaccharide or a derivative thereof, or a salt thereof.

4. The preservation solution according to any one of claims 1 to 3, wherein the preservation solution is an isotonic solution.

5. The preservation solution according to claim 4, wherein the isotonic solution is a lactated Ringer's solution.

6. The preservation solution according to any one of claims 3 to 5, wherein the polysaccharide is dextran.

7. The preservation solution according to any one of claims 1 to 6, wherein a concentration of the trehalose or derivative thereof, or salt thereof is 2.0 to 6.0% (w/v).

8. The preservation solution according to claim 6 or 7, wherein a concentration of the dextran or derivative thereof, or salt thereof is 4.0 to 7.0% (w/v).

9. The preservation solution according to any one of claims 1 to 8, wherein the preservation solution is for preserving a mammalian cell at 0 to 40°C.

10. The preservation solution according to any one of claims 1 to 9, wherein the preservation solution is for preserving a mammalian cell for a period of 6 h to 14 days.

11. The preservation solution according to any one of claims 1 to 10, wherein the preservation solution is used for suppressing a decrease in viability of a mammalian cell.

12. The preservation solution according to any one of claims 1 to 11, wherein the preservation solution is used for suppressing a decrease in self-renewal potential of a mammalian cell.

13. The preservation solution according to any one of claims 1 to 12, wherein the preservation solution is used for mammalian cell transplantation.

14. The preservation solution according to any one of claims 1 to 13, wherein the mammalian cell is a mammalian stem cell.

15. The preservation solution according to claim 14, wherein the mammalian stem cell is a mammalian mesenchymal stem cell.

16. A powder formulation comprising trehalose or a derivative thereof, or a salt thereof and a hydrogen carbonate as a pH modifier, for preparing the preservation solution according to any one of claims 1 to 15.

17. The powder formulation according to claim 16, wherein the hydrogen carbonate is sodium bicarbonate.

18. A method of preserving a mammalian cell, comprising a step of preserving a mammalian cell in a preservation solution comprising trehalose or a derivative thereof, or a salt thereof and a hydrogen carbonate as a pH modifier, and having a pH of 6.5 to 8.5.

19. The method of preserving a mammalian cell according to claim 18, wherein the hydrogen carbonate is sodium bicarbonate.

20. The method of preserving a mammalian cell according to claim 18 or 19, wherein the preservation solution further comprises a polysaccharide or a derivative thereof, or a salt thereof.

21. The method of preserving a mammalian cell according to any one of claims 18 to 20, wherein the preservation solution is an isotonic solution.

22. The method of preserving a mammalian cell according to claim 21, wherein the isotonic solution is a lactated Ringer's solution.

23. The method of preserving a mammalian cell according to any one of claims 20 to 22, wherein the polysaccharide is dextran.

24. The method of preserving a mammalian cell according to any one of claims 18 to 23, wherein the mammalian cell is preserved in the preservation solution for 6 h to 14 days.

[Fig. 1]

A

Cell viability (%) vs Preservation period (days)

- Example 3
- Example 2
- Example 1
- Comparative Example 3
- Comparative Example 2
- Comparative Example 1

B

Viable cell recovery rate (%) vs Preservation period (days)

- Example 3
- Example 2
- Example 1
- Comparative Example 3
- Comparative Example 2
- Comparative Example 1

[Fig. 2]

[Fig. 3]

[Fig. 4]

Mean±SD （n=4）

**: $p < 0.01$ vs CSP-01 (5.6 unadjusted)
Dunnet multiple comparison test

## INTERNATIONAL SEARCH REPORT

| | International application No. |
|---|---|
| | PCT/JP2020/017586 |

A. CLASSIFICATION OF SUBJECT MATTER
C12N 5/07(2010.01)i; C12N 1/04(2006.01)i
FI: C12N1/04; C12N5/07

According to International Patent Classification (IPC) or to both national classification and IPC

B. FIELDS SEARCHED

Minimum documentation searched (classification system followed by classification symbols)
C12N5/07; C12N1/04

Documentation searched other than minimum documentation to the extent that such documents are included in the fields searched

| Published examined utility model applications of Japan | 1922–1996 |
|---|---|
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)
JSTPlus/JMEDPlus/JST7580 (JDreamIII); CAplus/MEDLINE/EMBASE/BIOSIS/WPIDS
(STN)

C. DOCUMENTS CONSIDERED TO BE RELEVANT

| Category* | Citation of document, with indication, where appropriate, of the relevant passages | Relevant to claim No. |
|---|---|---|
| Y | WO 2014/208053 A1 (OTSUKA PHARMACEUTICAL FACTORY, INC.) 31.12.2014 (2014-12-31) claims, paragraphs [0020]–[0033] | 1–24 |
| Y | JP 2006-230396 A (NIPRO CORP.) 07.09.2006 (2006-09-07) claims, paragraph [0014], experiment example 3 | 1–24 |

| ☐ Further documents are listed in the continuation of Box C. | ☒ See patent family annex. |
|---|---|

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 06 July 2020 (06.07.2020) | 14 July 2020 (14.07.2020) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**
Information on patent family members

International application No.

PCT/JP2020/017586

| Patent Documents referred in the Report | Publication Date | Patent Family | Publication Date |
|---|---|---|---|
| WO 2014/208053 A1 | 31 Dec. 2014 | US 2016/0120170 A1 claims, paragraphs [0036]-[0048] EP 3015545 A1 | |
| JP 2006-230396 A | 07 Sep. 2006 | US 2006/0078872 A1 claims, paragraph [0022], experimental example 3 EP 1647186 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012115253 A
- WO 2014208053 A
- WO 9622362 A
- WO 02101057 A
- US 5843780 A
- US 6200806 B
- US 6280718 B

**Non-patent literature cited in the description**

- **GAGE, F.H.** *Science,* 2000, vol. 287, 1433-1438
- **MORRISON, S.J. et al.** *Cell,* 1999, vol. 96, 737-749
- **BATLE, E. et al.** *Cell,* 2002, vol. 111, 251-263
- **LANE, T.A. et al.** *Transfusion,* 2009, vol. 49, 1471-1481
- *Cell,* 1992, vol. 70, 841-847
- *Cell,* 2006, vol. 126, 663-676
- *Nature,* 2007, vol. 448, 313-317
- *Nat Biotechnol,* 2008, vol. 26, 101-106
- *Cell,* 2007, vol. 131, 861-872
- *Science,* 2007, vol. 318, 1917-1920
- *Cell Stem Cells,* 2007, vol. 1, 55-70
- *Nat Biotechnol,* 2007, vol. 25, 1177-1181
- *Nature,* 2007, vol. 448, 318-324
- *Cell Stem Cells,* 2008, vol. 2, 10-12
- *Nature,* 2008, vol. 451, 141-146
- *Nature,* 1997, vol. 385, 810
- *Science,* 1998, vol. 280, 1256
- *Nature Biotechnology,* 1999, vol. 17, 456
- *Nature,* 1998, vol. 394, 369
- *Nature Genetics,* 1999, vol. 22, 127
- *Proc. Natl. Acad. Sci. USA,* 1999, vol. 96, 14984
- *Nature Genetics,* 2000, vol. 24, 109
- *Journal of Autoimmunity,* 2008, vol. 30, 163-171